# EUROPEAN PATENT SPECIFICATION

(11) **EP 2 376 461 B1**
(45) Date of publication and mention of the grant of the patent: **11.03.2015**
(21) Application number: 10702454.9
(22) Date of filing: 15.01.2010
(51) Int. Cl.: A61K 31/4245

(54) **NITRIC OXIDE FUROXAN DERIVATIVE COMPOUNDS ENDOWED WITH ANTITUMORAL ACTIVITY**
STICKOXIDFUROXANDERIVATVERBINDUNGEN MIT ANTITUMORAKTIVITÄT
COMPOSÉS DÉRIVÉS DE FUROXANE LIBÉRANT DE L'OXYDE NITRIQUE DOTÉS D'UNE ACTIVITÉ ANTITUMORALE

(30) Priority: 15.01.2009 WO PCT/EP2009/000206
(43) Date of publication of application: 19.10.2011
(73) Proprietor: Humanitas Mirasole S.p.A., 20089 Rozzano (IT); Istituto Oncologico Veneto Irccs, 35128 Padova (IT)
(72) Inventor: VIOLA, Antonella, I-20089 Rozzano (IT); BRONTE, Enzo, I-35128 Padova (IT); CROSETTI, Marco, I-20089 Rozzano (IT); LAZZARATO, Loretta, I-20089 Rozzano (IT); FRUTTERO, Roberta, I-20089 Rozzano (IT); GASCO, Alberto, I-20089 Rozzano (IT)
(74) Representative: Long, Giorgio
(86) International application number: PCT/EP2010/050455
(87) International publication number: WO 2010/081877

(56) References cited:
- WO-A-2004/096793
- DE-A1- 4 420 523

## Description

### FIELD OF THE INVENTION

The present invention pertains to the field of medicine; in particular, it concerns new furoxan derivative compounds able to inhibit some metabolic pathways involved in tumoral development.

### BACKGROUND

Prostate cancer is the second leading cause of malignancy-related mortality in males in the Western world. While radical prostatectomy and local radiotherapy are largely successful for patients with localized cancer, available treatments for metastatic prostate carcinoma (PCa) have demonstrated weak curative efficacy. It is therefore necessary to find alternative therapeutic approaches to hormone-refractory metastatic prostate cancer. Immunotherapy may provide valid alternative therapy for patients with hormone-refractory metastatic PCa. The success of this approach depends on the ability of cytotoxic T cells to kill tumor cells. However, if the tumor environment exerts a suppressive action on antigen-specific tumor infiltrating lymphocytes (TIL), immunotherapy will achieve little, if any, successes. Thus, it is paramount to understand the cell biology of TIL and the modulation of TIL responses by the tumor environment.

The role of the prostate tumor environment in modulating T cell response have been analyzed with a study based on the use of collagen gel-matrix supported organ cultures of human PCa. The advantage of using this technique is that the microenvironment remains intact and all the factors that may affect TIL functions, such as cell-cell interaction, cell-matrix supported interaction and interstitial fluid, are preserved. This innovative approach to TIL biology allowed to obtain several important new findings.

In principle, TIL infiltrating PCa samples are mainly CD8+ T lymphocytes with an antigen-experienced, terminally differentiated phenotype (CD8+ CD45RA+ CD62L-CCR7-), positive for perforin and therefore able to kill the cancerous cells; however, they are in dormant state since they do not express activation markers such as CD25, CD69 and CD137. Moreover, different from normally responsive lymphocytes in tumor free prostates and peripheral blood, TIL are not activated locally by powerful signals acting either on TCR or downstream signaling pathways, indicating a tumor-restricted deficiency. In addition, evidence has been accumulating that arginase (ARG) and nitric oxide synthase (NOS) enzymes are over expressed in PCa as compared with hyperplasic prostate, with the intriguing observation that the tumor cells themselves rather than myeloid infiltrating cells could be the main source of the enzymes. The results indicate that the steady-state regulation of the dormant state is dependent on the enhanced intra-tumoral metabolism of the amino acid L-Arginine (L-Arg), since the simple addition of ARG and NOS specific inhibitors was sufficient to rouse these CTL, activate them and start a number of events leading to cytolitic granule polarization and killing of cognate targets. In addition, it has also been demonstrated the presence of high levels of nitrotyrosines in TILs, suggesting a local production of peroxynitrites, possibly due to ARG and NOS activities, since by inhibiting the activity of the enzymes a reduced tyrosine nitration was also achieved.

These results identify a mechanism by which human prostate cancer induces *in situ* immunosuppression. Thus, drug controlling the generation of reactive nitrogen species (RNS) might be useful to aid immunotherapeutic approches for the treatment of cancer, by creating a favorable tumor environment for lymphocyte activation (Bronte et al., 2005).

Results from clinical trials have shown that the efficacy of different immunotherapeutic approaches is not adequate for an immediate and widespread transfer of this novel therapeutic approach to cancer patients. An important emerging concept is that the altered metabolism present in tumor microenvironment may have a profound impact on antitumor activity. Considering results mentioned above, it is clear that drugs controlling the generation of reactive nitrogen species (RNS) might enhance significantly the impact of immunotherapeutic approaches for the treatment of cancer. Accordingly, the present invention provides new furoxan compounds acting on the mechanisms of tumoral development.

### SUMMARY OF THE INVENTION

Considering the strict relationship between ARG, NOS and cyclooxigenase enzymes, all over-expressed in prostate cancer cells as compared to normal prostate epithelium, the present invention concerns novel small molecules interfering with multiple and interconnected metabolic pathways. In contrast to conventional adjuvants, such as cytokines and activators of antigen presenting cells, which are characterized by broad action on the immune system, lack of selectivity and potentially important side effects, the molecules of the present invention potentiate the function of effector anti-tumor lymphocytes either spontaneously present, elicited in patients upon vaccination, or injected through an adoptive immunotherapy protocol.

### OBJECT OF THE INVENTION

In first object, the present invention concerns N-oxides of 1,2,5-oxadiazole as per claims 1.

In a second embodiment, the present invention relates to the compounds of the invention for use as a medicament as per claim 2 and, as a preferred embodiment, to their use as medicaments for the therapy of pathologies characterized by the generation of reactive nitrogen species, according to claim 3 and 4.

As a preferred embodiment, they are for use for the treatment of prostatic cancer, as per claim 5.

As a third embodiment, the invention relates to a pharmaceutical preparation comprising the compounds of the invention, according to claim 7 and 8.

In a further embodiment, the present invention relates to the use of the compounds of the invention as adjuvant in the immunotherapeutic protocols against cancer as per claim 6.

### BRIEF DESCRIPTION OF THE FIGURES

FIG. 1 shows the results of the proliferation assay.
FIG. 2 shows the results of the chromium assay.
FIG. 3 shows a graph of the immunohistochemal analysis.
FIG. 4 shows the results of the in vivo experiments on compound AT38.

### DETAILED DESCRIPTION OF THE INVENTION

In a first embodiment, the present invention relates to new furoxan compounds.

In particular, the compounds of the invention are those of the following formulae:

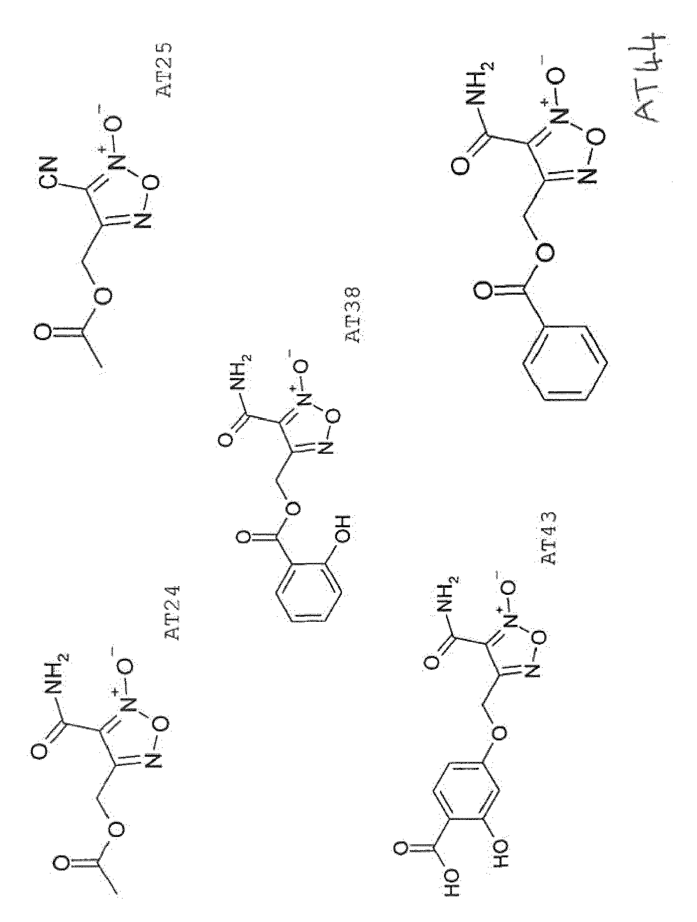

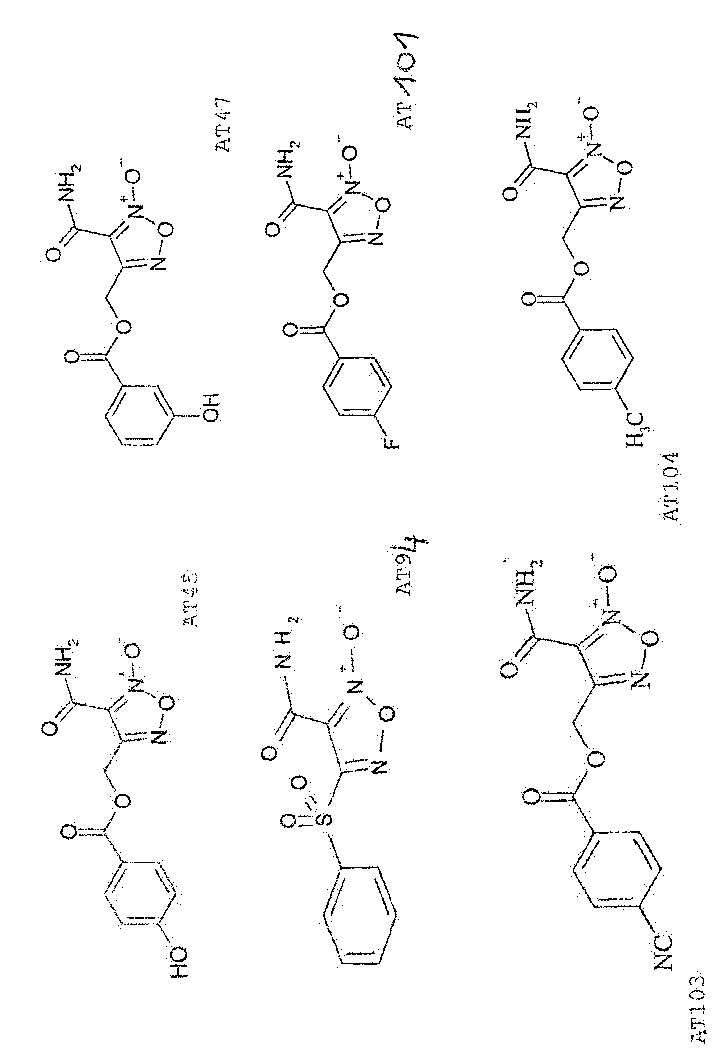

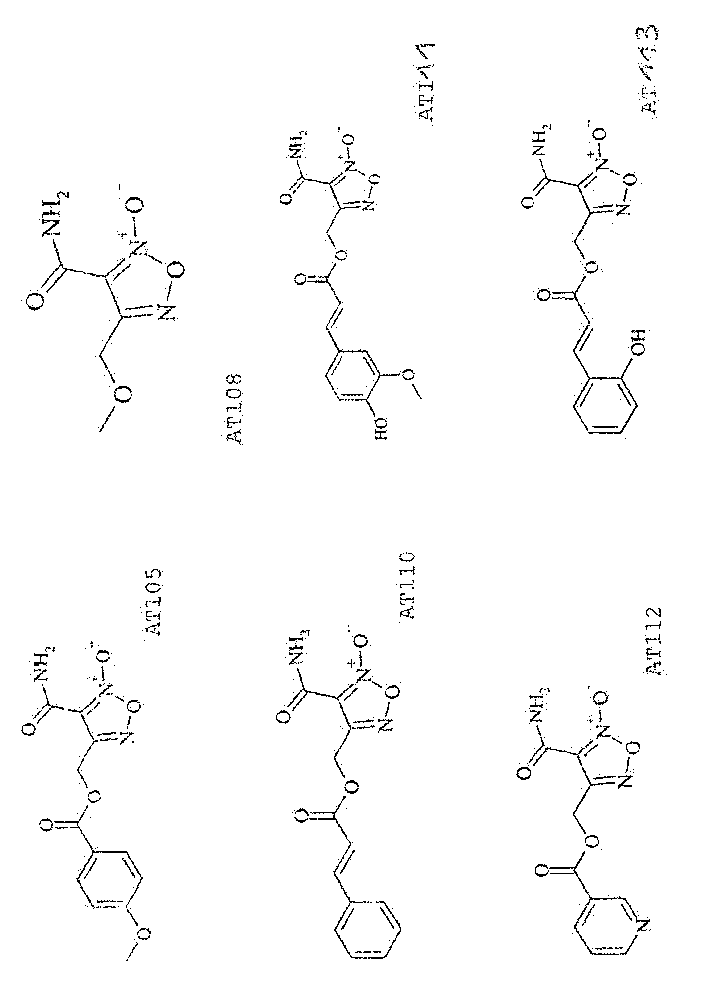

### Synthesis of the selected compounds of the invention

### General procedure for preparation of benzoic esters of 4-(hydroxymethyl)furoxan-3-carboxamide.

To a stirred suspension of KF (3 mmol) and 4-(bromomethyl)furoxan-3-carboxamide (1 mmol) in dry DMF (10 mL), the appropriate benzoic acid (1 mmol) was added and the mixture of reaction was stirred at RT for 1 h.

Then, water (30 mL) was added and resulting mixture was extracted with EtOAc (3x10 mL). The combined organic phases were washed with 10 % NaHCO₃ solution (2x10 mL), brine (1x10 mL), and then dried on MgSO₄. Solvent was removed under reduced pressure to give a white solid that was purified by flash-cromatography using the reported eluents.

Analytical samples were obtained by crystallization with appropriate solvents.
1) **[3-(aminocarbonyl)furoxan-4-yl]methyl benzoate**
   Eluent: PE 7 / EtOAc 3; yield: 80%.
   M.p.: 163-165 °C (H₂O / iPrOH).
   ¹H-NMR (DMSO-d₆): δ, 8.51 (*s*_{br}, 1H, -CON*H*₂); 8.01 (d, 2H, Ph); 7.82 (*s*_{br}, 1H, -CON*H*₂); 7.70 (t, 1H, Ph); 7.56 (t, 2H, Ph); 5.63 (*s*, 2H, -OC*H₂*-Fx).
   ¹³C-NMR (DMSO-d₆): δ, 164.9; 155.6; 154.7; 133.7; 129.2; 128.9; 128.7; 110.3; 57.4.
   Mass (CI) (m/z): 264 [MH⁺].
   Anal. Calcd. for C₁₁H₉N₃O₅ C% 50.19, H% 3.45, N% 15.96; found C% 50.44, H% 3.58, N% 16.04.
**2) [3-(aminocarbonyl)furoxan-4-yl]methyl salicylate**
   Eluent: CH₂Cl₂ 8 / EtOAc 2; yield: 80%.
   M.p.: 176-177 °C (H₂O / iPrOH).
   ¹H-NMR (CDCl₃): δ, 8.51 (*s*_{br}, 1H, -CON*H*₂); 7.86 (d, 1H, Ph); 7.59 (*s*_{br}, 1H, -CON*H₂*); 7.53 (t, 1H, Ph) ; 6.95 (m, 2H, Ph); 5.70 (*s*, 2H, -OC*H*₂-Fx).
   ¹³C-NMR (DMSO-d₆): δ, 167.0; 159.8; 155.6; 154.0; 135.8; 130.2; 119.2; 117.6; 112.7; 110.4; 57.5.
   Mass (CI) (m/z): 280 [MH⁺].
   Anal. Calcd. for C₁₁H₉N₃O₆ C% 47.32, H% 3.25, N% 15.05; found C% 47.36, H% 3.58, N% 14.97.
**3) [3-(aminocarbonyl)furoxan-4-yl]methyl** 3-**hydroxybenzoate**
   Eluent: CH₂Cl₂ 8 / EtOAc 2; yield: 82%.
   M.p.: 203-204 °C (H₂O / iPrOH).
   ¹H-NMR (DMSO-d₆): δ, 9.91 (s, 1H, Ph-O*H*); 8.51 (*s*_{br}, 1H, -CON*H*₂); 7.83 (*s*_{br}, 1H, -CON*H*₂); 7.46-7.31 (m, 3H, Ph); 7.09-7.06 *(m,* 1H, Ph); 5.61 *(s,* 2H, -OC*H*₂-Fx).
   ¹³C-NMR (DMSO-d₆): δ, 130.6; 165.7; 158.3; 156.4; 155.5; 130.7; 121.6; 120.9; 116.6; 111.2; 60.5.
   Massa (CI) (m/z): 280 [MH⁺].
   Anal. Calcd. for C₁₁H₉N₃O₆ C% 47.32, H% 3.25, N% 15.05; found C% 46.97, H% 3.18, N% 15.45.
**4) [3-(aminocarbonyl)furoxan-4-yl]methyl 4-hydroxybenzoate**
   Eluent: CH₂Cl₂ 8 / CH₃OH 2; yield: 50%.
   M.p.: 241-242 °C (H₂O /iPrOH).
   ¹H-NMR (DMSO-d₆): δ, 10.44 (*s*, 1H, Ph-O*H*); 8.49 (*s*_{br}, 1H, -CON*H*₂); 7.85 (d, 2H, Ph) ; 7.82 (*s*_{br}, 1H, -CON*H*₂); 6.87 (d, 2H, Ph); 5.55 (s, 2H, -OC*H*₂-Fx).
   ¹³C-NMR (DMSO-d₆): δ, 164.7; 162.4; 155.6; 154.9; 131.7; 119.2; 115.4; 110.3; 57.0.
   Mass (CI) (m/z): 280 [MH⁺].
   Anal. Calcd. for C₁₁H₉N₃O₆ C% 47.32, H% 3.25, N% 15.05; found C% 47.29, H% 3.25, N% 15.17.
5) **[3-(aminocarbonyl)furoxan-4-yl]methyl 4-fluorobenzoate**
   Eluent: CH₂Cl₂ 8 / EtOAc 2; yield: 90%.
   M.p.: 163-164 °C (H₂O / iPrOH).
   ¹H-NMR (DMSO-d₆): δ, 8.52 (*s_{br}*,, 1H, -CONH₂); 8.08 *(m,* 2H, Ph) ; 7.83 (*s_{br}*,, 1H, -CONH₂); 7.37 (t, 2H, Ph); 5. 62 (s, 2H, - OC*H*₂-Fx).
   ¹³C-NMR (DMSO-d₆): δ, 165.3 (d, J¹_{CF} = 270 Hz); 164.0; 155.6; 154.6; 132.3 (d, J³_{CF} = 9. 75 Hz); 125.3; 116.0 (*d,* J²_{CF} = 22.2 Hz) ; 110. 4; 57.6.
   Mass (CI) (m/z): 282 [MH⁺].
   Anal. Calcd. for C₁₁H₈N₃O₅F C% 46.98, H% 2.87, N% 14.94; found C% 47.10, H% 2.86, N% 15.00.
**6) [3-(aminocarbonyl)furoxan-4-yl]methyl 4-nitrobenzoate**
   Eluent: CH₂Cl₂ 9 / EtOAc 1; yield; 86%.
   M.p.: 172-173 °C (H₂O / iPrOH).
   ¹H-NMR (DMSO-d₆): δ, 8.53 (*s_{br},* 1H, -CON*H*₂); 8.28 (d, 2H, Ph); 8.25 (d, 2H, Ph); 7.85 (*s_{br},* 1H, -CON*H₂*); 5.76 (s, 2H, -OC*H*₂-Fx).
   ¹³C-NMR (DMSO-d₆): δ, 163.5; 155.4; 154.3; 150.4; 134.1; 130.8; 123.9; 110.4; 58.1.
   Mass (CI) (m/z): 309 [MH⁺]
   Anal. Calcd. for C₁₁H₈N₄O₇ C% 42.87, H% 2.62, N% 18.18; found C% 42.85, H% 2.67, N% 18.05.
**7) [3-(aminocarbonyl)furoxan-4-yl]methyl 4-cyanobenzoate**
   Eluent: CH₂Cl₂ 9 / EtOAc 1; yield: 87%.
   M.p.: 190-191 °C (CHCl₃ / *n*-Hex).
   ¹H-NMR (DMSO-d₆): δ, 8.51 (*s_{br},* 1H, -CON*H₂*); 8.15 (d, 2H, Ph); 8.14 (d, 2H, Ph); 7.82 (*s_{br}*, 1H, -CON*H*₂); 5.66 (s, 2H, -OC*H*₂-Fx).
   ¹³C-NMR (DMSO-d₆): δ, 163.7; 155.5; 154.4; 132.9; 132.6; 129.9; 117.9; 115.8; 110.4; 57.9.
   Mass (CI) (m/z): 289 [MH⁺].
   Anal. Calcd. for C₁₂H₈N₄O₅ C% 50.01, H% 2. 79, N% 19.44; found C% 50.13, H% 2.84, N% 19.48.
**8) [3-(aminocarbonyl)furoxan-4-yl]methyl** 4-**methylbenzoate**
   Eluent: CH₂Cl₂ 9.5 / EtOAc 0.5; yield: 68%.
   M.p.: 149-150 °C (H₂O / iPrOH).
   ¹H-NMR (DMSO-d₆): δ, 8.51 (*s_{br}*, 1H, -CON*H*₂); 7.90 (d, 2H, Ph); 7.84 (*s_{br}*, 1H, -CON*H*₂); 7.37 (d, 2H, Ph); 5.61 (s, 2H, -OC*H*₂-Fx); 2.41 (s, 3H, Ph-C*H*₃).
   ¹³C-NMR (DMSO-d₆): δ, 164.8; 155.5; 154.7; 144.2; 129.4; 129.3; 125.9; 110.3; 96.9; 57.3.
   Mass (CI) (m/z):278 [MH⁺].
   Anal. Calcd. for C₁₂H₁₁N₃O₅ C% 51.98, H% 3.99, N% 15.16; found C% 52.10, H% 3.99, N% 15.14.
**9) [3-(aminocarbonyl)furoxan-4-yl]methyl** 4-**methoxybenzoate**
   Eluent: CH₂Cl₂ 9 / EtOAc 1; yield: 95%.
   M.p.: 175-176 °C (H₂O / iPrOH).
   ¹H-NMR (DMSO-d₆): δ, 8.50 (*s_{br}*, 1H, -CON*H*₂); 7.94 (d, 2H, Ph) ; 7.83 (*s_{br}*, 1H, -CON*H*₂); 7.06 (d, 2H, Ph) ; 5.83 (s, 2H, -OC*H*₂-Fx); 3.85 (*s*, 3H, -OC*H*₃).
   ¹³C-NMR (DMSO-d₆): δ, 164.5; 163.5; 155.6; 154.8; 131.5; 120.8; 114.1; 110.3; 57.2; 55.5.
   Mass (CI) (m/z) : 294 [MH⁺].
   Anal. Calcd. for C₁₂H₁₁N₃O₆ C% 49.15, H% 3.78, N% 14.33; found C%49.27, H% 3.79, N% 14.33.
**10) [3-(aminocarbonyl)furoxan-4-yl]methyl 4-aminobenzoate**
   Eluent: CH₂Cl₂ 9 / EtOAc 1; yield: 89%.
   M.p.: 206-208 °C (H₂O / iPrOH).
   ¹H-NMR (DMSO-d₆): δ, 8.50 (*s_{br},* 1H, -CON*H*₂); 7.83 (*s_{br}*, 1H, -CON*H*₂); 7.69 (d, 2H, Ph) ; 7.59 (d, 2H, Ph); 6.09 (s, 2H, -NH₂); 5.52 (s, 2H, -OC*H*₂-Fx).
   ¹³C-NMR (DMSO-d₆): δ, 164.9; 155.6; 155.2; 153.9; 131.4; 114.4; 112.6; 110.3; 56.6.
   Mass (CI) (m/z): 279 [MH⁺].
   Anal. Calcd. for C₁₁H₁₀N₄O₅ C% 47.48, H% 3.62, N% 20.14; found C% 47.31, H% 3.60, N% 20.20.
**11) [3-(aminocarbonyl)furoxan-4-yl]methyl 4-chlorobenzoate**
   Eluent: CH₂Cl₂ 9 / EtOAc 1; yield: 98%.
   M.p.: 171-172 °C (n-Hex / iPrOH).
   ¹H-NMR (DMSO-d₆): δ, 8.51 (*s_{br},* 1H, -CON*H*₂); 8.02 (d, 2H, Ph); 7.83 (*s_{br}*, 1H, -CON*H*₂); 7.65 (d, 2H, Ph) ; 5. 63 (s, 2H, -OC*H*₂-Fx).
   ¹³C-NMR (DMSO-d₆): δ, 164.1; 155.5; 154.5; 138.7; 130.8; 129.0; 127.6; 110.4; 57.7.
   Mass (CI) (m/z): 298 [MH⁺].
   Anal. Calcd. for C₁₁H₈N₃O₅Cl C% 44.38, H% 2.71, N% 14.12; found C% 44.32, H% 2.75, N% 14.13.

It is submitted that the person skilled in the art will be able to preparare other compounds falling within the scope of the present invention without undue burden.

### MATERIALS AND METHODS

### Cell lines and mice.

CT26 (H-2d), a BALB/c carcinogen-induced colon carcinoma; MBL-2 (H-2b), a Moloney virus-induced lymphoma; C26-GM, a cell line derived from the C26 colon carcinoma (H-2d) genetically modified to release granulocyte-macrophage colony-stimulating factor (GM-CSF).

Cells were grown in DMEM (Invitrogen) or in RPMI medium 1640 (Euroclone) supplemented with 2 mM L-glutamine, 10 mM Hepes (DMEM) or 1 mM sodium pyruvate (RPMI 1640), 20 mM 2-mercaptoethanol, 150 units/ml streptomycin and 200 units/ml penicillin, 10% heat-inactivated FBS (Invitrogen or BioWhittaker).

BALB/c (H-2^{d}) and C57BL/6 (H-2^{d}) mice (8 weeks old) were purchased from Harlan.

BALB/c mice were inoculated s.c. in the inguinal fold with 0.5 x10⁶ C26GM cells. Mice were killed after 9 days and splenocytes were used for *in vitro* assay.

For *in vivo* experiments, BALB/c mice were inoculated s.c. on the left flank with 0.5 x10⁶ C26GM cells.

### Proliferation assay

BALB/c splenocytes from control animals and from coloncarcinoma26 (C26GM) tumor bearing-mice were plated at 6x10⁵ cells/well and stimulated with 3 µg/ml anti-CD3 (2C11, ATCC) and 2 µg/ml anti-CD28 (clone 37.5, ATCC) either with or without scalar dilutions of each furoxan derivate as adjuvant. After 2 days of incubation, 1 µCi/well (1 Ci=37 GBq) of ³H-TdR (PerkinElmer) was added to the cultures for 18 h, and ³H-TdR incorporation was measured by scintillation counting.

### Chromium release assay

Two different cell cultures were set up to evaluate the CTL response. First, BALB/c splenocytes (6x10⁵ cells/well) were stimulated with (6x10⁵ cells/well) γ-irradiated C57BL/6 splenocytes in 96-well, flat-bottom plates (BD Falcon), either with or without furoxan derivates at scalar dilutions. To obtain immunosuppression, CD11b⁺ cells sorted from the spleens of tumor-bearing mice were added at a final concentration of 3% to a mixed leucocytes culture. Second, immunosuppressed splenocytes (6x10⁵ cells/well) derived from tumor-bearing mice were stimulated with γ-irradiated C57BL/6 splenocytes (6x10⁵ cells/well) in 96-well, flat-bottom plates either with or without furoxan derivates at scalar dilutions. The percentage of CD11b⁺ cells present in the spleen of these mice varied from 20 to 40%.

In both experimental conditions, after 5 days of incubation, cultures were tested for ability to kill 2x10³ allogenic (MBL-2) or singenic (CT26) target cells in a 5-h ⁵¹Cr-release assay.

The percentage of specific lysis was calculated from triplicate samples as follows: (experimental cpm-spontaneous cpm)/(maximal cpm-spontaneous cpm) x100, whereas litic unit 30 (LU30) represent the number of CTL cells required to kill 30% of target cells.

### Immunohistochemistry

The tumors were fixed in PLP fixative (Paraformaldehyde/Lysine/Periodate), cryoprotected in 30% sucrose and frozen in OCT. The samples were cut with a cryostat (6 mm) and after air drying, the sections were fixed with acetone for 3 min. Subsequently, the slides were rehydrated with PBS and endogenous peroxidase activity and the aspecific sites were blocked. The tissue sections were incubated with the primary antibodies anti-Nitrotyrosine (1:200, Calbiochem) or anti-CD3 (1:50, Dako) for 2h at r.t. After washes with PBS, the samples were incubated with goat-anti-rabbit-peroxidase (Dako) for 1h at r.t. Immunoreactivity was visualized with 3,3-diaminobenzidine (DAB). Sections were counterstained with hematoxylin and mounted in Eukitt.

### Adoptive cell therapy

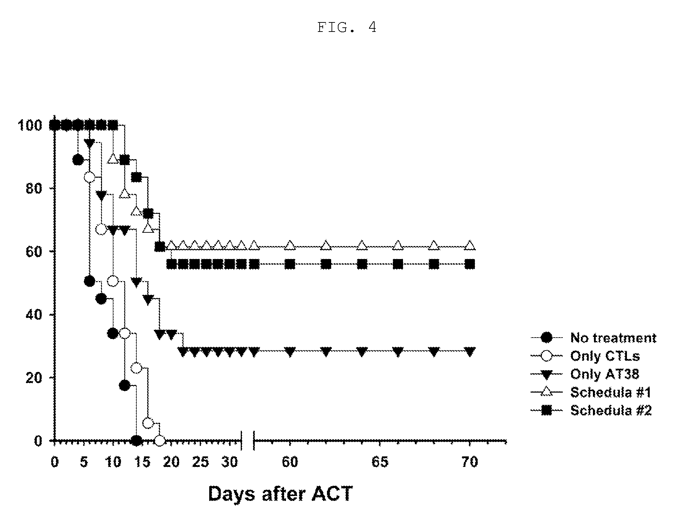

Adoptive cell therapy (ACT) was performed after inoculation of C57BL/6 mice with 10⁵ EG-7 tumor cells subcutaneously on day 0. Tumor was allowed to grow and, at day 6, drug treatment was started. Mice were divided in 5 groups: tumor growth control (receiving only carboxymethylcellulose); adoptive transfer of tumor-antigen specific CTLs; AT38 alone; schedule 1 (mice were treated continuously after day 6 till sacrifice) together with adoptive transfer; schedule 2 (drug was suspended the day after cells transfer) and adoptive cell transfer. Treated mice received AT38 at 30 mg/kg/die divided in 2 doses of 15mg/kg every 12 hours. On day 10, 5x10⁶ tumor antigen-specific CTLs were injected i.v. and mice were then treated i.p. with 30,000 IU of recombinant IL-2 twice a day for 3 consecutive days. On day 14 mice were sacrificed and tumors were taken for immunohistochemical analysis. Other mice were followed for survival as shown in Fig. 4.

### RESULTS

**Table 1**

| | ***in vitro* lympho-proliferation** | ***in vitro* cytotoxicity assay** | |
|---|---|---|---|
| | **Range of concentration to rescue T cell proliferation (µM)** | **Range of concentration to rescue cytolytic activity (µM)** | |
| **COMPOUNDS** | **C26GM spleen** | **B6+C26GM** | **B6+Balb+ 3%CD11b+** |
| AT24 | 100-12.5 | INEFFECTIVE | INEFFECTIVE |
| AT25 | 25-6.25 | INEFFECTIVE | not yet tested |
| AT27 | 63-13 | 25 | 63-32 |
| AT38 | 72-18 | 25 | 72-34 |
| AT43 | 68 | INEFFECTIVE | 68-17 |
| AT44 | 76-38 | INEFFECTIVE | 76 |
| AT45 | 72-9 | 100 | 72 |
| AT47 | 72-18 | 50 | 72-18 |
| AT84 | 100 | 50-25 | 50-25 |
| AT88 | 19-6.25 | 1.25 µg/ml | 6.25-3.1 |
| AT94 | 19-9 | INEFFECTIVE | 10-5 |
| AT101 | 285-142 | 100-50 | not yet tested |
| AT103 | 140 | 100-50 | not yet tested |
| AT104 | 140 | 100-50 | not yet tested |
| AT105 | 273-68 | 100-50 | not yet tested |
| AT108 | 1.8X10³ | Not yet tested | not yet tested |
| AT110 | 200-100 | 100 | not yet tested |
| AT111 | 100 | 100-50 | not yet tested |
| AT112 | 200 | 200 | not yet tested |
| AT113 | 50 | 100 | not yet tested |

Table 1 above reports a partial list of compounds that have been screened. The second column from left reports the minimal effective concentration of each furoxan adjuvant required to restore the T cell proliferation in immunosuppressive condition. Twenty out of 95 adjuvants restored T lymphocytes responsiveness, which was inhibited by the presence of myeloid suppressor cells.

Adjuvants, which demonstrate to be ineffective in a proliferation assay were discarded, while the others were tested in a cytotoxicity assay to evaluate their efficacy in restoring T cell cytolytic activity against allogenic target cells using two different immunosuppressive conditions (third and fourth column from left).

Nine out of twenty compounds completely restored the cytolitic function of CD8⁺ T cells in the alloreactive cultures containing 3% of immunosuppressive CD11b⁺ cells, one is ineffective (fourth column).

Under strong immunosuppressive conditions (third column) 14 of 20 compounds restored the alloreactivity of CD8⁺ T cells, whereas 5 of 20 were ineffective.

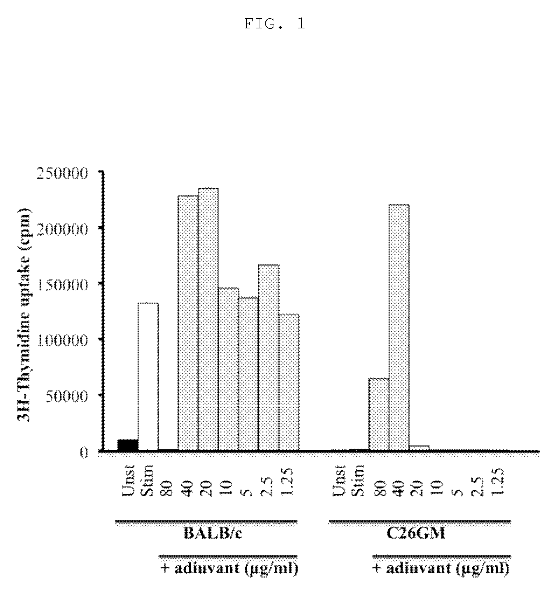

Figure 1 shows the results of one representative assay among those performed (results for other 19 compounds were similar). In particular, it can be seen that adjuvant used in this assay restored T lymphocytes responsiveness inhibited by the presence of myeloid suppressor cells. No toxic effects were observed on control cell coltures by the furoxan derivate since scalar dilution of each compound does not affect lymphocyte proliferation of control BALB/c cell coltures(left panel), whereas restored the proliferation under immunosuppressive conditions (right panel).

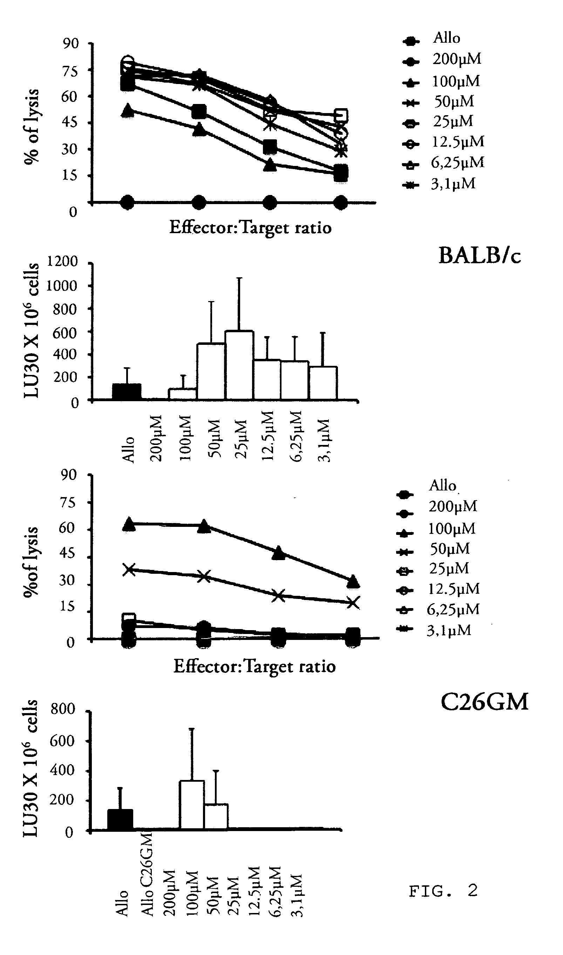

Figure 2 depicts the results of a representative chromium release assay. A single dose of each compound was added at time 0 to the alloantigen-stimulated T cell coltures. In the upper panel, scalar dilutions of the furoxan derivate do not to affect the cytolitic activity of BALB/c control cell coltures except at the higher dose (200 µM). Under immunosuppressive conditions, 100 µM and 50 µM of furoxan derivate are able to restore the cytolytic functions of CD8⁺ T cell as shown by LU30/10⁶ cells that is comparable to the control. No effect was seen at lower concentrations. These data are representative of one compound, the other 19 showing similar results.

We also evaluated the solubility in physiological vehicles and the stability at pH 2 for 24 h in order to understand if we could perform an oral-administration of the adjuvants.

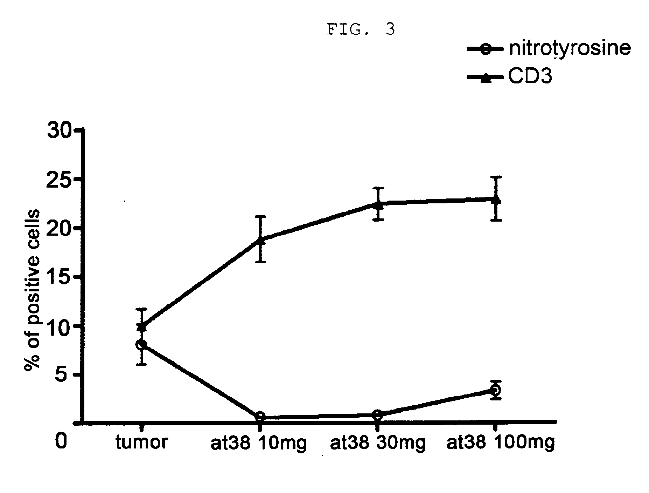

Based on the findings, it was investigated whether the compound AT38 could be useful in antitumor immunity elicited by cancer vaccination in tumor-bearing mice. The animals were injected subcutaneously with coloncarcinoma26 at day 0. The animals were treated for nine days with 3 different doses of AT38 (10, 30 and 100 mg/kg/day) orally administered starting from day 0 (see Fig. 3). At day 10 the animals were euthanized and tumors removed for immunohistochemistry analysis. Compared to untreated controls, animals that have received AT38 for nine days showed an enhancement of CD3⁺ T tumor infiltrating lymphocytes and a strong reduction of nitrotyrosine staining.

According to the above, it is a second object of the invention the compounds of the invention for use as a medicament.

In a preferred embodiment, they are used for the treatment of pathologies characterized by the generation of RNS (reactive nitrogen species), such as, for instance, neoplasia, inflammatory diseases or chronicle infections.

In a still preferred embodiment, the compounds of the invention may be used for the treatment of prostate cancer.

As per the above, it is a third object of the invention, the disclosed compounds for use as adjuvant in the immunotherapeutic protocols against the above mentioned pathologies and, in particular, against malignant cancer.

For the purposes of the present invention, the disclosed compounds may be formulated, together with excipients and additives selected in the group comprising diluent, solvents, bulking agents, fillers, reological modifier, stabilizers, binders, lubricants, disintegrant, preservatives, pH adjusting agents, buffers, antioxidant, chelating agents, plasticizer, polymers, emulsifiers, edulcorants, flavoring agents; alone or in combination thereof, to give a pharmaceutical preparation as per the third embodiment of the invention. In particular, "pharmaceutically acceptable salt" is intended to include any salts suitable to be administered to human or animal and having suitable technological properties, such as, for instance, sodium, potassium, ammonium, zinc salt or any salts with amino acids (see, for a general reference, Remington's Pharmaceutical Sciences Handbook, Mack Pub. Co., N.Y., USA 17th edition, 1985).

## Claims

1. Compounds selected among the following:

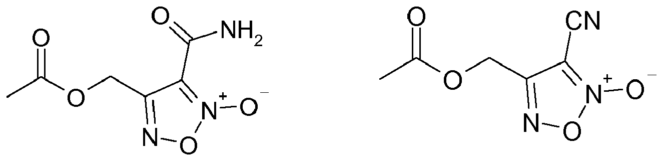

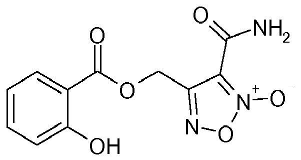

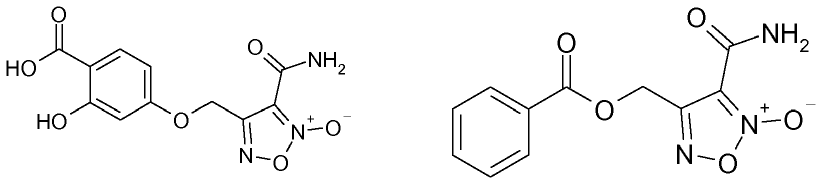

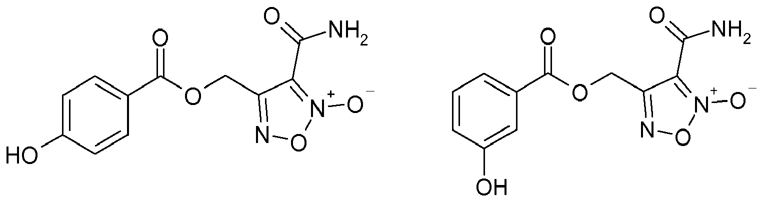

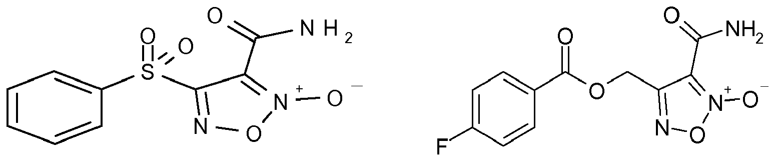

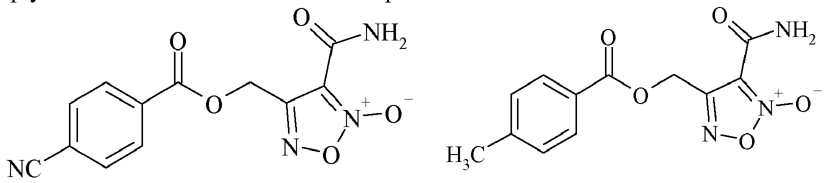

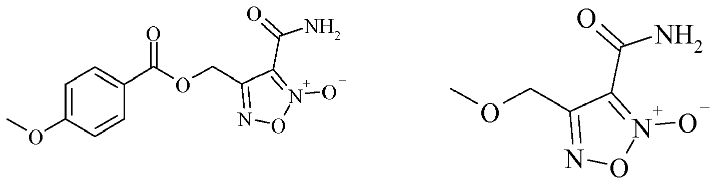

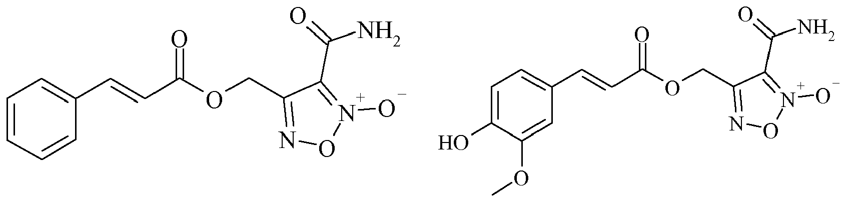

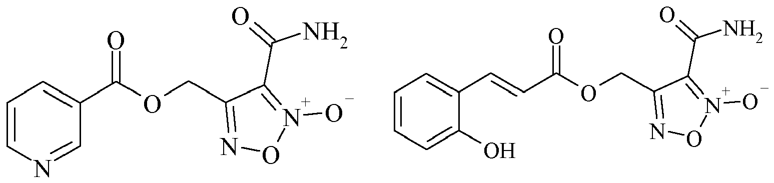

2. Compounds according to claims 1 for use as a medicament.

3. Compounds according to claim 1 or 2 for use as a medicament for the therapy of pathologies **characterized by** the generation of reactive nitrogen species.

4. Compounds for use according to claim 3, wherein said pathologies comprise neoplasia, inflammatory diseases or chronicle infections.

5. Compounds for use according to claim 4 for the treatment of prostate cancer.

6. Compounds for use according to claim 5 as adjuvant in the immunotherapeutic protocols against cancer.

7. A pharmaceutical preparation comprising one or more of the compounds of claim 1.

8. The pharmaceutical preparation of claim 7 further comprising excipients and additives selected in the group comprising diluent, solvents, bulking agents, fillers, rheological modifier, stabilizers, binders, lubricants, disintegrant, preservatives, pH adjusting agents, buffers, antioxidant, chelating agents, plasticizer, polymers, emulsifiers, edulcorants, flavoring agents; alone or in combination thereof.

## Patentansprüche

1. Verbindungen ausgewählt aus den Folgenden:

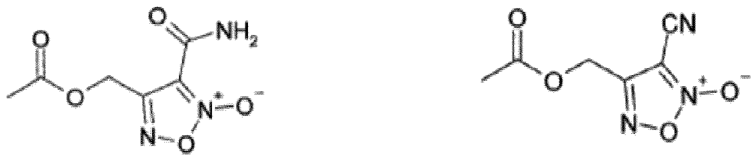

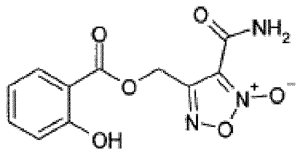

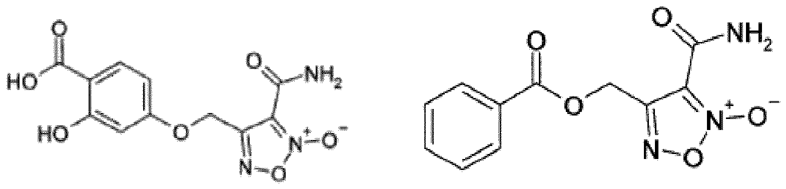

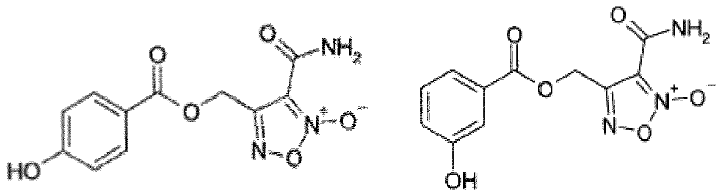

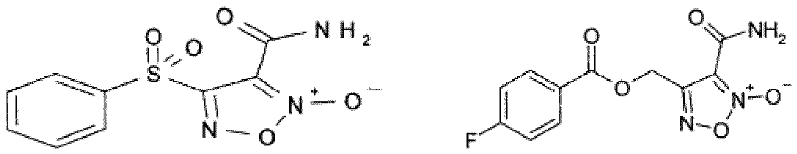

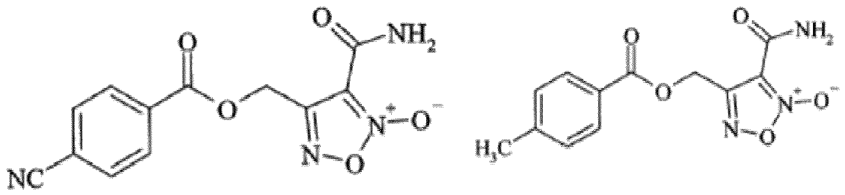

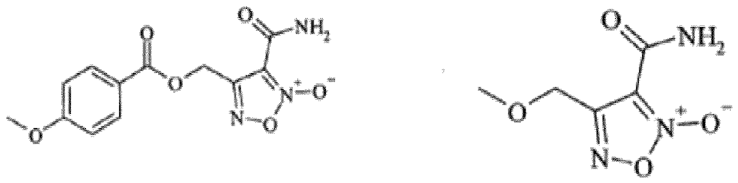

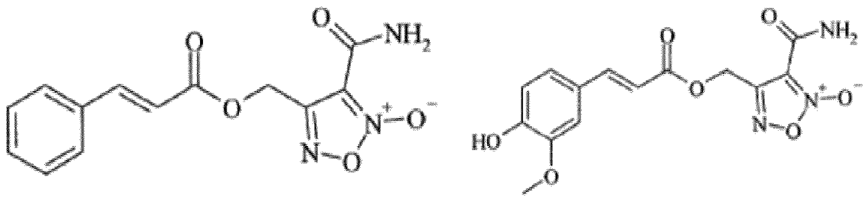

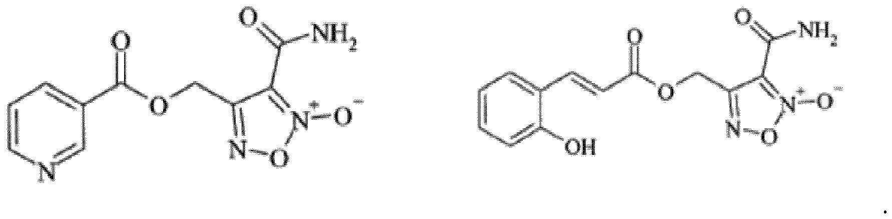

2. Verbindungen nach Anspruch 1 zur Verwendung als ein Medikament.

3. Verbindungen nach den Ansprüchen 1 oder 2 zur Verwendung als ein Medikament zur Behandlung von Pathologien, die **gekennzeichnet sind durch** die Erzeugung von reaktiven Stickstoff-Verbindungen.

4. Verbindungen zur Verwendung nach Anspruch 3, wobei die Pathologien Neoplasie, entzündliche Krankheiten oder chronische Infektionen umfassen.

5. Verbindungen zur Verwendung nach Anspruch 4 zur Behandlung von Prostata Krebs.

6. Verbindungen zur Verwendung nach Anspruch 5 als Begleittherapie in den immunotherapeutischen Protokollen gegen Krebs.

7. Pharmazeutische Zubereitung, die ein oder mehr der Verbindungen von Anspruch 1 umfasst.

8. Pharmazeutische Zubereitung nach Anspruch 7, die weiter Hilfsstoffe und Zusätze umfasst, die ausgewählt werden aus der Gruppe umfassend Verdünnungsmittel, Lösungsmittel, Füllstoffe, Füller, rheologische Modifikatoren, Stabilisatoren, Bindemittel, Gleitmittel, Sprengmittel, Konservierungsmittel, pH einstellende Mittel, Puffer, Antioxidant, Chelatbildner, Weichmacher, Polymere, Emulgatoren, Süßstoffe, Aromastoffe; allein oder in Kombination davon.

## Revendications

1. Composés choisis parmi les suivants :

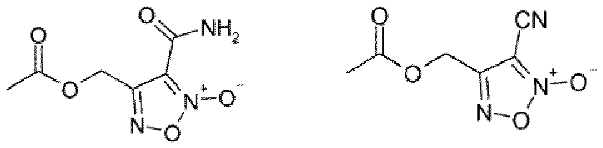

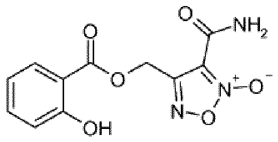

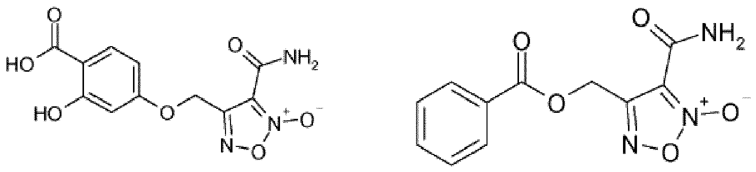

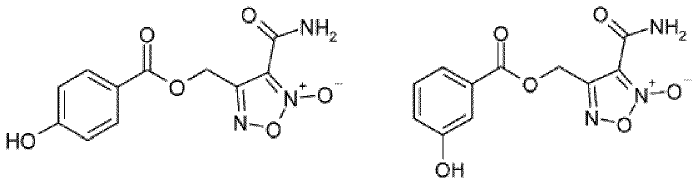

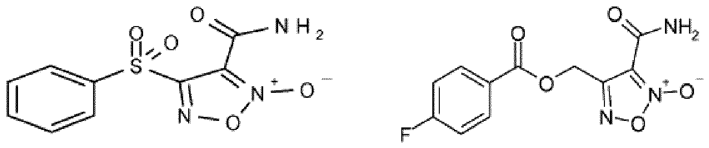

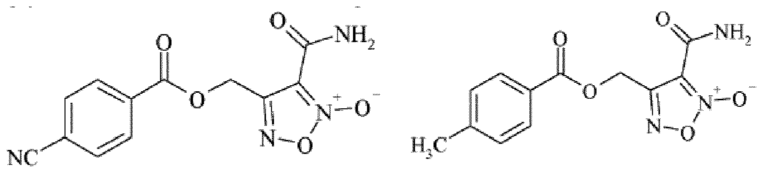

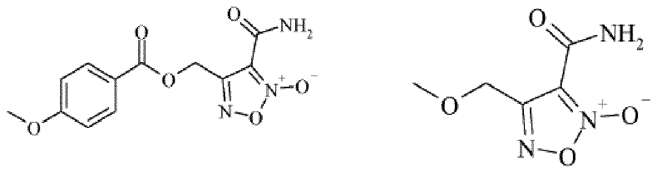

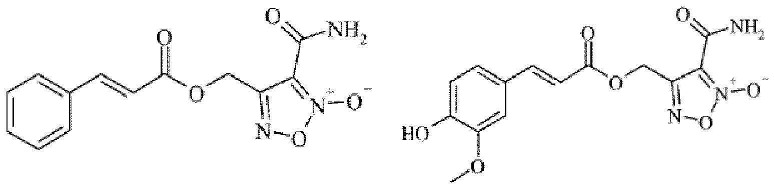

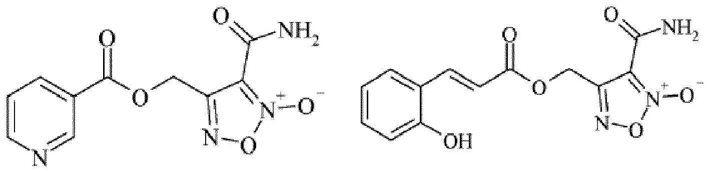

2. Composés selon la revendication 1 destinés à être utilisés comme médicament.

3. Composés selon la revendication 1 ou 2 destinés à être utilisés comme médicament pour la thérapie de pathologies **caractérisées par** la production d'espèces d'azote réactives.

4. Composés destinés à être utilisés selon la revendication 3 où lesdites pathologies comprennent la néoplasie, les maladies inflammatoires ou les infections chroniques.

5. Composés destinés à être utilisés selon la revendication 4 pour le traitement du cancer de la prostate.

6. Composés destinés à être utilisés selon la revendication 5 comme adjuvant dans les protocoles immunothérapeutiques contre le cancer.

7. Préparation pharmaceutique comprenant un ou plusieurs des composés de la revendication 1.

8. Préparation pharmaceutique selon la revendication 7 comprenant en outre des excipients et des additifs choisis dans le groupe comprenant un diluant, des solvants, des agents conférant du volume, des charges, un modificateur rhéologique, des stabilisants, des liants, des lubrifiants, un désintégrant, des conservateurs, des agents d'ajustement du pH, des tampons, un antioxydant, des agents chélateurs, un plastifiant, des polymères, des émulsifiants, des édulcorants, des agents aromatisants; seuls ou dans une combinaison de ceux-ci.
